# EUROPEAN PATENT SPECIFICATION

(11) **EP 0 721 331 B1**
(45) Date of publication and mention of the grant of the patent: **05.12.2001**
(21) Application number: 94929076.1
(22) Date of filing: 29.09.1994
(51) Int. Cl.: A61K 9/16, A61K 9/72, B01J 2/00

(54) **PROCESS I**
VERFAHREN I
PROCEDE (I)

(30) Priority: 01.10.1993 SE 9303214; 22.12.1993 SE 9304271
(43) Date of publication of application: 17.07.1996
(73) Proprietor: AstraZeneca AB, 151 85 Södertälje (SE)
(72) Inventor: TROFAST, Eva, S-226 47 Lund (SE); FALK, Johan, S-223 68 Lund (SE)
(86) International application number: PCT/SE94/00896
(87) International publication number: WO 95/09615

(56) References cited:
- EP-A- 0 072 046
- EP-A- 0 291 201
- GB-A- 2 187 952
- US-A- 4 161 516
- US-A- 5 143 126

## Description

The invention relates to a method and an apparatus for making a powder consisting of particles having a particle size smaller than 10µm free-flowing by forcing the particles to create agglomerates.

Powders consisting of very small particles are commonly used in the inhalation therapy where the size of the particles are of utmost importance. The diameter of particles which are to be inhaled must be less than 10 µm to ensure the adequate penetration of the particles into the bronchial area of the lungs.

Most finely divided powdered medicament, such as micronized powders, are light, dusty and fluffy and they often create problems during handling, processing and storing. For particles having a diameter less than 10 µm the van der Wahls forces are generally greater than the force of gravity and consequently the material is cohesive. The particles tend to adhere to each other forming non-defined agglomerates. Such powders have very poor free-flowing properties which often make handling and precise metering problematic.

One possible method of making these powders free-flowing or at least improve their flowing properties is to force, in a controlled manner, the primary particles to form larger particles, agglomerates. Non-defined agglomerates are formed at random when this finely divided powdered medicament is handled, for instance during storage, conveying, sieving, sifting, mixing or grinding.

It is common knowledge that spherical agglomerates flow freely, packs easily and uniformly, have an ideal form for coatings and are therefore commonly used in drug formulations.

The flow of very cohesive powders can be improved by vibration-induced agglomeration. Depending on the type of powder, liquid (often water) or solid binders are added during the agglomeration, but it is also possible to agglomerate without binders.

The method of agglomeration is applicable in principle to all materials, including mixtures of various powders. Any powder, provided it is fine enough, can be granulated or pelletized without a binder by systematic agitation or rolling of the particulate material.

The common principle of performing this agglomeration is to let the individual particles be exposed to a systematic movement of a powder bed, without changing the physical and chemical properties of the primary particles.

The agglomerated powders consist of relatively large, more dense and compact spheres which exhibit the normal flow properties, but at the same time the spheres should have sufficient low internal coherence to break up into small primary particles of medicament of a therapeutically effective size during inhalation in an inhalation device.

The inhaled route of administration enables the dose to be delivered directly to the airways. By this type of administration it is possible to give a small dose and thereby minimizing unwanted side effects which for example could occur if the substance ends up in another part of the body, e.g. the gastrointestinal tract or the oro-laryngeal tract.

Methods of controlled agglomeration are known in the prior art. For example, Claussen and Petzow (Journal of Materials Technology, vol 4(3), 148-156 (1973)) have described a dry agglomeration method where no binders are intentionally added for preparation of spheres in the size range 0.1 - 3 mm by tumbling in a cylinder tilted at an angle to the horizontal axis of rotation. According to the authors a dry agglomeration process for small particles requires nuclei in order to start, but almost all powders consist of natural agglomerates that function as nuclei. They also conclude that agglomerates from commonly used devices such as a rotating drum or a granulating pan form a wide size distribution of the agglomerates that makes frequent sieving necessary. The product formed often exhibit bad sphericity and low density.

US-A-5 143 126 describes a vibratory conveyor for forming flowable agglomerates from previously poorly flowable fine-grained powder by using a method wherein the poorly flowable powder is subjected to a mechanical vibration step prior to transport and metering.

GB-A-1 569 611 describes a process for agglomeration of a drug into soft pellets. In this process moist is used as a binder to provide a doe which by extrusion is pressed through a sieve to create agglomerates.

GB-A-2 187 952 describes a method in which crystalline ibuprofen is compacted by kneading as it is conveyed by conveying screws through an extruder. The obtained agglomerates can also be passed through an extruder plate affixed to the end of the extruder.

It is an object of the present invention to provide a method of controlled agglomeration of finely divided powdered medicament having a primary particle size smaller than 10 µm, preferably smaller than 5 µm, for example such as micronized powders, in which no binders are needed and in which the obtained agglomerates are of a uniform size having a structure which provides sufficient flowability for the transport and metering of such powders and which nevertheless have sufficient low internal coherence to break up, within an inhalation device, such as a dry powder inhalator, into particles of medicament of a therapeutically effective size, e.g. having a particle size which is smaller than 10 µm.

The method according to the invention provides a process for facilitating the technical handling and significantly increase the medical value of the substance. It has been found that this method produces agglomerates having excellent handling properties, which have sufficient strength to withstand packaging and storage, but which are sufficiently soft so that they will break down into primary particles when they are expelled from an inhalator during inhalation therapy.

According to the present invention there is provided a method for the manufacture of agglomerates, which comprises subjecting the finely divided particles of the medicament, which could be in admixture with any other ingredient desired to be incorporated into the agglomerates, to mechanical unit operations under certain conditions. More specifically there is provided a method of treatment of a finely divided powdered medicament having a particle size smaller than 10 µm and poor flowing properties to form, in a controlled manner, agglomerates or pellets which are free flowing and which are capable of breaking down to provide the finely divided medicament, comprising the steps of:
a) agglomerating the powdered medicament having particles sizes being smaller than 10µm by feeding the material to a screw feeder, letting the material pass through the screw feeder thereby obtaining agglomerates,
b) spheronizing the resulting agglomerates in order to provide agglomerates which are more spherical, more dense and more compact than the agglomerates obtained from the agglomeration process in the screw feeder,
c) sizing the agglomerates to obtain a uniform size of the final product.

According to the invention there is also provided an apparatus for performing the method of treatment of a finely divided powdered medicament having a particle size smaller than 10 µm and poor flowing properties to form, in a controlled manner, agglomerates or pellets which are free flowing and which are capable of breaking down to provide the finely divided medicament, wherein the apparatus comprises a screw feeder with at least two co-operating screws which are rotating, a spheronizing device for spheronizing the resulting agglomerates and a sizing device for sizing the agglomerates to obtain a uniform size of the final product.

Further preferred steps of the method are dear from the appended dependent claims 2 - 10 and preferred embodiments of the apparatus are clear from the appended dependent claims 11 - 16.

There is also provided a use of the apparatus to carry out the method according to the invention.

It is also an object of the invention to provide a use of the agglomerates manufactured in accordance with the method in a breath actuated dry powder inhaler, such as Turbuhaler® .

The agglomeration of the powder is now described with reference to the drawings which show a prefered embodiment of the apparatus as well as diagrams from the results of experiments made in accordance with the invention, wherein
Fig. 1 shows a schematic view of a first embodiment of an apparatus according to the invention,
Fig. 2a shows a schematic view of the screws in the screw feeder,
Fig. 2b shows a schematic view of the screws mounted within the housing,
Fig. 3 shows a schematic view of a second embodiment of an apparatus according to the invention,
Fig. 4a and 4b show the differences in agglomerates obtained from a screw feeder having long-pitch screws and short-pitch screws respectively,
Fig. 5 is a diagram showing a comparison of the size of the agglomerated particles as a function of different screws,
Fig. 6 is a diagram showing size distribution of spheres as a function of different screws,
Fig. 7 is a diagram of a sieving analysis of micronised terbutaline sulphate processed in an apparatus according to the invention.

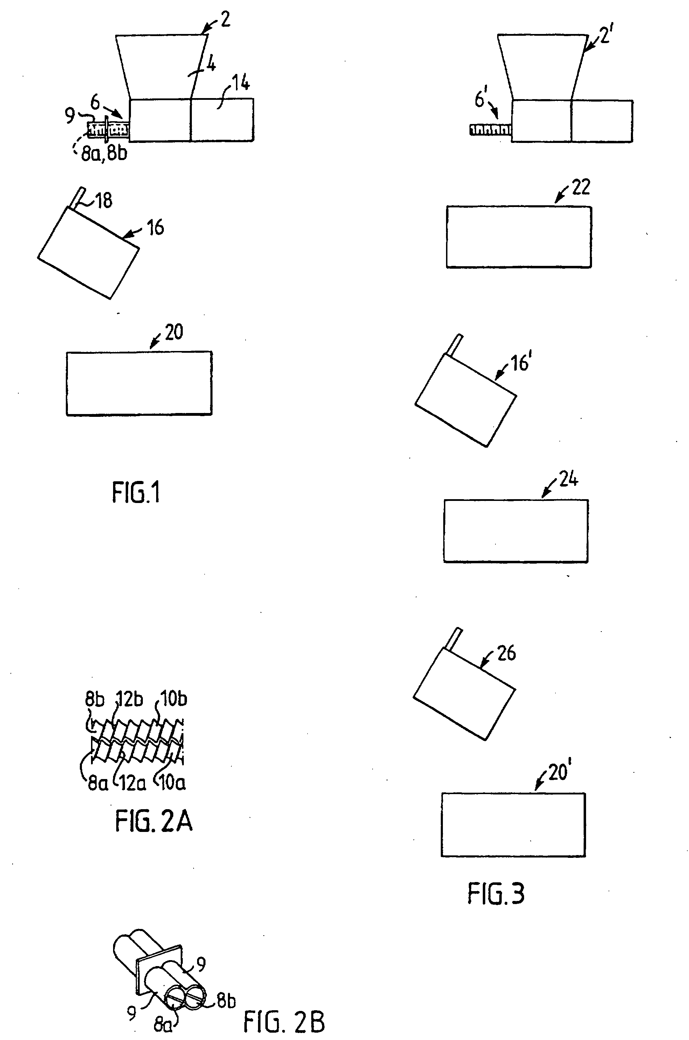

The apparatus used to carry out the method according to the invention is shown in fig.1. The apparatus includes an agglomerator in the form of a screw feeder apparatus 2 comprising a receiving vessel 4 and a screw feeder device 6. The screw feeder device 6 comprises at least two screws 8a, 8b being surrounded by a housing 9. The finely divided powdered medicament is supplied to the screw feeder device 6 through the vessel 4. The vessel 4 is provided with a mechanical stirring device (not shown) to facilitate the feeding of the cohesive powder to the screws. The stirring device could be of any known type, for example having L-shaped arms extending form a shaft provided perpendicular to the vertical axis of the container.

The powder is transported through the screw feeder device 6 and due to the pressure which is created between the at least two screws 8a, 8b by the twisting movement of the screws 8a, 8b, which is created by a motor 14, the powder particles will be pressed together and form soft agglomerates of different sizes. The agglomerated particles which are obtained from the agglomeration process in the screws 8a, 8b of the screw feeder 6 have a size between 0.1 mm - 2 mm, are flowable, due to their size, and comparatively soft in their structure.

A screw feeder device suitable to be used for the agglomeration according to the invention is a device having so-called Twin-concave screws whereby the screws have identical pitches. The agglomeration procedure of the finely divided powdered medicament is obtained as the powder is mechanical forced into the grooves 10a, 10b between the pitches 12a, 12b of the co-operating screws 8a, 8b as they rotate. As the screws rotate the pitches 12a of one screw 8a will move into the groove 10b of the other screw 8b and thereby removing attached powder on the screw and forcing the powder forward at the same time in a form of cleaning process. In this manner the cleaning process of the screws will create agglomerates of the powder which is forced into the distance between the screws, see especially fig.2a.

Tests have shown that Twin-concave screws having a short pitch will give the most uniform agglomerates having the best properties for meeting the requirements for powders for inhalation. The tests have also shown that the length and the rotation speed of the screws are of minor importance for the result of the agglomeration procedure and that mainly the interval of the pitch of the screws and the groove between the pitches are of importance. More dense and uniform agglomerates are obtained from screws where the distance between the pitches of the two screws is as small as possible. The measure between the outer diameter and the inner diameter of the screws should be between 1 - 10 mm, preferably between 1 - 5 mm. If this distance is too big the agglomerates will not have the well defined dimensions which are required. In a preferred embodiment the outer diameter of the screws is 20 mm and the inner diameter of the screws is between 10 - 19 mm, preferably between 15 - 19 mm. It is also important for the characteristics of the obtained agglomerates that the housing 9 around the screws in the screw feeder fits around the screws in a tight manner leaving only a minimal groove between the walls of the housing and the screws, see fig. 2b. If there is a distance between the wall and the screws finely divided powdered medicament will be compacted in this area during the rotation of the screws and the agglomeration procedure will result in a less uniform product

In a preferred embodiment of the invention the pitches of the screws are between 2 - 20 mm, preferably between 5 - 15 mm. Suitable screw feeders include the K-TRON SODER standard twin shaft feeder without agitator and the Brabender twin-screw feeder type DDSR/20.

After the agglomeration procedure the agglomerates can be transported to a sieving device in order to obtain agglomerates within a certain range of size if desired.

The agglomerates obtained from the screw feeders have different sizes and are comparatively soft and need to be further treated to obtain the desired characteristics. The agglomerates are therefore collected in a spheronizing device, preferably a rotating container, such as a pan or drum 16 and which is preferably provided with one or more scrapers 18 (only shown schematically in the drawings). The container 16 is tilted and rotating. The rotating movement of the container 16 will make the agglomerates rotate and tumble due to the tilting of the container. During the rotation the agglomerates will obtain a stronger, more spherical, dense, compact and uniform form and a smoother outer surface. The characteristics achieved in the rotating container will further improve the flowability and the resistance against breaking during handling and storage. The speed of the container determines the characteristics of the agglomerates after this spheronization. Tests have shown that the optimal periphery speed of the container is between 0.2 - 2.0, preferably between 0.4 - 1.0 m/s. The spheronization time is preferably between 1 - 20 min. Tests have shown that after 3 - 10 min the agglomerates often have obtained required optimal size, capability of breaking down to provide the finely divided medicament and density for their future use. These characteristics are, as already mentioned above, of utmost importancy when the agglomerates are to be used in the inhalation therapy.

Tests have shown that the most optimate tilting angle of the container 16 is between 10° - 80° from the vertical, preferably between 30° - 60° as an angle chosen herebetween gives the best densifying and growing effect to the agglomerates.

The granulating container is made of a material which is inert and do not contaminate the powder, such as for example metal, plastic or any other suitable material. In order to avoid electrostatic forces to build up during the spheronization process the container could be grounded.

After the spheronization in the tilted container 16 the agglomerates are supplied to a sieve 20 having an aperture size which is between 0.2 - 2.0 mm, preferably between 0.3 - 1.0 mm. The sieving is used in order to obtain a uniform size of the agglomerates. The requirements for the use of this operation is strongly depending on the type of inhalation device to be used.

The requirements of uniform size and proper density is higher if the agglomerates are to be used in a dry powder inhaler or in a metered dose inhaler. During inhalation it is of utmost importance that agglomerates are broken up into a high amount of primary particles having a particle size smaller than 10 µm.

To utilize the process according to the invention in the most efficient and economic manner and to minimize the amount of agglomerates which are too big and therefore have to be recycled into the process it would be advantageous to incorporate further steps of sieving and spheronization into the process. Tests have shown that the most efficient manner to carry out the agglomeration process according to this invention is to incorporate two further steps of sieving and one further step of spheronization. A further sieving step is hereby incorporated into the process directly after the agglomeration process in the screw feeder. After this sieving the agglomerates are spheronized in the granulating container and a second further sieving step is carried out after this spheronization. A second spheronization step is then carried out and the whole process is ended by the final sieving step. These further steps of sieving and spheronization will provide a more effective process and the agglomerates obtained after the second spheronization are uniform and have the required characteristics.

An apparatus according to this embodiment of the invention is shown in fig. 3.

According to this figure the finely divided powdered medicament is agglomerated in the screw feeder device 6' and the resulting agglomerates are supplied to a sieve 22. The sifted agglomerates are thereafter supplied to the tilted granulating container 16'. After the spheronization in the container 16' the agglomerates are supplied to a second sieve 24 to obtain a more uniform size. After this second sieving the agglomerates are spheronized a second time in a second tilted granulating container 26. This second granulating container 26 is of the same type as the first container and the periphery speed and the spheronization time are defined above for the first step of spheronization. After this second spheronization the agglomerates are sifted through the final sieve 20' to obtain a uniform size of the final product. The sieving after the spheronization is necessary as in some case the agglomerates might grow too much during the spheronization and therefore the final product could contain agglomerates having a size being bigger than the required size, e.g. 0.2 - 2 mm, preferably 0.3 - 1 mm.

The agglomerates obtained from the process according to the invention are to be used in a dry-powder inhalator, and preferably in a dry-powder breath-actuated inhalator. The hardness of the agglomerates are therefore of utmost importancy. The required hardness of agglomerates which are capable of breaking down into primary particles during inhalation has been measured by a MHT-4 Microhardness tester. (A.Paar, Austria) and has been found to vary between 0.5 to 20 mN for agglomerates having good deagglomeration properties and which break down into the required primary particles in an inhalator during inhalation. With values above 20 mN the deagglomeration of the agglomerates will be less and above 100 mN very little deagglomeration of the agglomerates will occur.

The agglomeration process according to the invention will now be described by experiments which are intended to illustrate but not limit the scope of the invention as described in the appended claims.

### Example 1

Properties of agglomerates of three different powders have been determined and is to be seen in the table below. The powder consisted of finely divided particles which was passed through the steps of the method according to the invention:

| SUBSTANCE | MASS MEDIUM DIAMETER (µm) | SURFACE AREA (m²/g) | BULKDENSITY (g/ml) |
|---|---|---|---|
| Terbutaline | 1.7 | 9 | 0.25 |
| Budesonide | 2.0 | 6 | 0.24 |
| Lactose | 3.0 | 6 | 0.32 |

Typically the bulk density for agglomerated powders consisting of finely divided particles can be determined to vary between 0.2 mg to 0.4 g/ml for particles having a particle size of less than 10 µm. The surface area varies between substances but there is no difference between micronised and micronised and agglomerated (and spheronised) powder. The area is between 2 - 20 m²/g, preferably 3 - 12 m²/g.

### Example 2

In order to examine the form of the agglomerates obtained from the method according to the invention a high-speed video camera was installed at the end of the screws. It was then possible to visualize the resulting product from the screw feeder and compare the formed agglomerates with non-treated powder under different experimental conditions. Samples was taken and was further examined under a microscope.

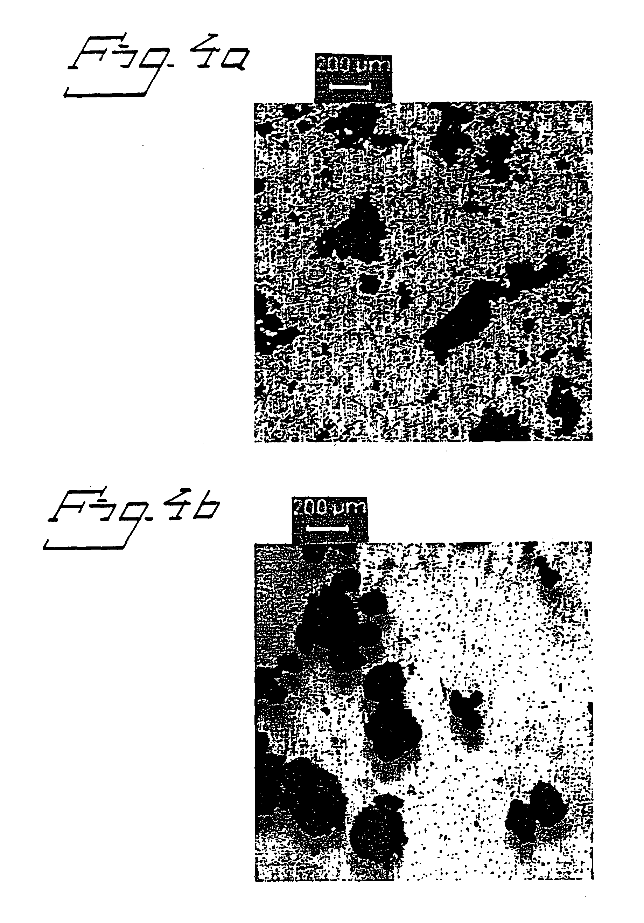

In the experiment micronised terbutaline sulphate with a mass medium diameter (MMD) of 1.2 µm was added to a K-tron Soder twin-shaft feeder (speed 15 gram/min) using long-pitch twin concave-profile screws. The resulting agglomerates were collected and examined under a microscope. Fig. 4a shows a picture of the irregular and soft agglomerates which was obtained.

In a further experiment the long-pitch screws were changed to short-pitch twin concave-profile screws of the same type and the same substance was added to the same equpiment and under the same experimental conditions. Fig 4b shows a picture of the agglomerates which are more regular.

### Example 3

The importance of the dimension of the screws was further established in order to examine the size of the agglomerates and especially the uniformity of the agglomerates - an important parameter for dosing accuracy. The results dearly indicate the necessity of using well-controlled agglomerating procedures.

In the experiments a Brabender twin-screw feeder type DDSR/20 was used. Micronised lactose (MMD < 4 µm) was added to the feeder with different twin-screw sizes (20/11, 20/12, 20/20; (length of pitch according to Brabender technical data sheet)). The soft agglomerates from the feeder was added to a gyro-vibrating sieve with a net size of 0.5 mm. The velocity of feeding on the sieve was adjusted so as the powder passed the sieve at once, i.e. no agglomeration was to occur on the sieve. Two experiments for each screw were performed. The result shows that screws with longer pitch (e.g. 20/20) results in less defined and larger agglomerates. As a comparative experiment micronised powder was slowly added by a spoon directely on the sieve, i.e. without passing the screw feeder. Great variation of the size of the agglomerates was obtained, which indicates the necessity of using a screw feeder to obtain more regular agglomerates and a satisfactory yield.

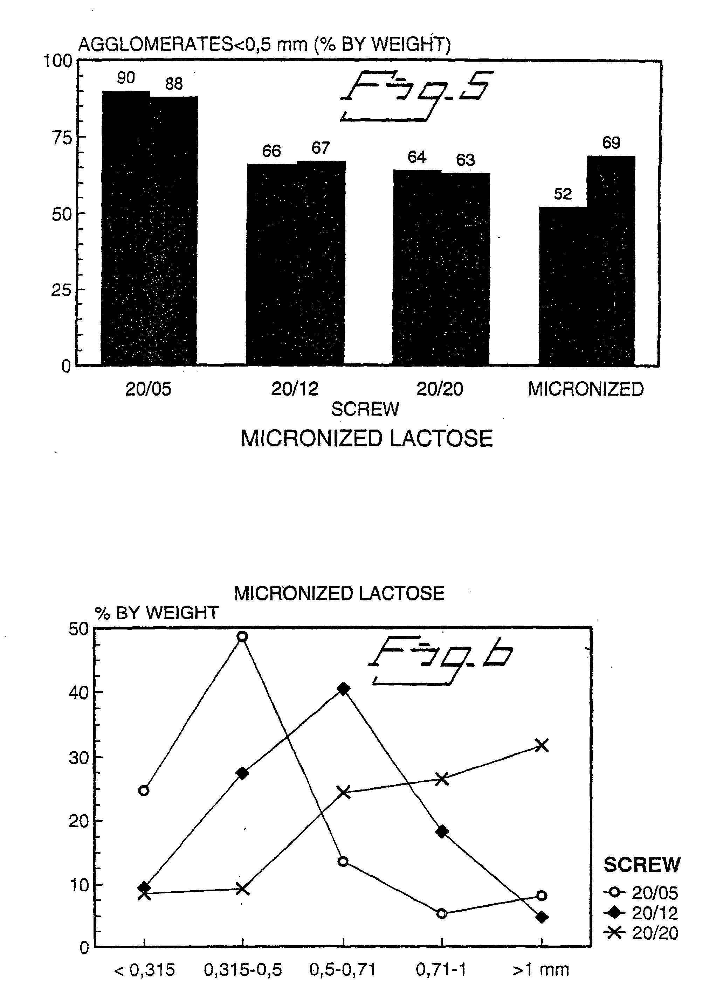

A summary of these experiments is to be seen in fig. 5. The reproducibility of the formation of the agglomerates and their properties like size, bulk density and distribution is thereby obtained by the present invention, which is of utmost importance for the accuracy of a dose, something which is especially important when the agglomerated and spheronized powder is to be used in a volume dosing inhalation device.

### Example 4

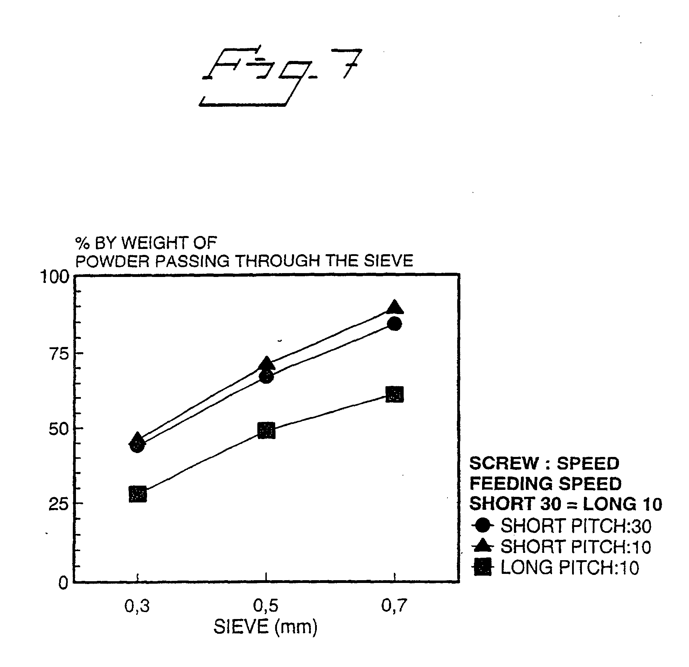

In order to determine the size of the agglomerates as a function of the screw dimension the following measures were taken. The agglomerates obtained in a Brabender twin-screw feeder type DDSR/20 were rotated at 35 rpm in a pan (diameter 320 mm) for 5 min. The agglomerates formed were sized in different fractions (< 0.315, 0.315-0.5, 0.5-071, 0.71-1 and >1 mm) by passing a gyro-vibrating sieve (Russel Finex). This sieving shows that the size of the spheres is strongly depending on the size and form of the screws. The final hardness of the agglomerates is determined by the spheronization procedure. The results are shown in fig. 6.

### Example 5

In a further experiment it was determined that the size of the agglomerates depends less on the speed of the screws than the size of the screws. Micronised terbutaline sulphate (MMD 1.2 µm) was fed to a K-Tron twin-screw feeder with different screws and speeds. The feeding speed 30 for the short pitch screws is equal to 10 for the long pitch screws (15 gram/min). The agglomerates were sized through 3 net dimensions (0.3, 0.5, 0.7 mm). The result is shown in fig. 7.

### Example 6

In a further experiment it was shown that by getting a uniform size of the agglomerates resulting from the agglomeration process in the screw feeder, the yield will increase in the subsequent steps as well as a more narrow distribution range of spheres will be obtained. This is further improved by using the multistep procedure as shown in fig. 3. In the experiment micronised terbutaline sulphate was agglomerated and spheronized. The resulting agglomerates were thereafter sized into different fraction and each fraction was filled into a powder inhaler. The metered dose was therafter determined for each fraction. The results shown in the diagram below which shows the relationship between different fractions of the substance and the metered dose give about 20% difference in dose when using different agglomerate sizes which dearly indicates the necessity of using a uniform size of the agglomerates in order to obtain a constant dose and small batch to batch variations.

| SIZE FRACTION | METERED DOSE (mg/dose) |
|---|---|
| < 0.14 | 0.65 |
| 0.14 - 0.3 | 0.62 |
| 0.3 - 0.5 | 0.59 |
| > 0.5 | 0.55 |

The agglomeration process described in the present invention gives a high yield of the total operation and acceptable batch to batch variations to the final product.

### Possible modifications

The method as well as the apparatus according to the invention could of course be modified within the scope of the appended claims.

Thus the construction and the size of the screw feeders as well as the speed and the length of the screws could be modified. The size of the apertures in the sieves which have been used could of course also be modified. Additional screws may also be used in the same manner.

It is also possible to modify the size, shape, speed and tilting angle of the granulating container thereby changing the size of the final agglomerates.

The spheronization could also be done in a so called marumerizer which is a commercially available apparatus for spheronization or granulation. The spheronization could also be done in any other suitable way using a rotation symmetrical receptade or container, which could be rotated, such as any container being cylindrical or barrel formed.

## Claims

1. Method of treatment of a finely divided powdered medicament having a primary particle size smaller than 10 µm and poor flowing properties to form, in a controlled manner, agglomerates or pellets which are free flowing and which are capable of breaking down to provide the finely divided medicament, comprising the steps of
a) agglomerating the powdered medicament having particles sizes being smaller than 10µm of the substance by feeding the material to a screw feeder, letting the material pass through the screw feeder thereby obtaining agglomerates,
b) spheronizing the resulting agglomerates in order to provide agglomerates which are more spherical, more dense and more compact than the agglomerates obtained from the agglomeration process in the screw feeder,
c) sizing the agglomerates to obtain a uniform size of the final product.

2. Method according to claim 1,
**characterised in that** a tilted granulating container, preferably with one or more scrapers, is used to spheronize the agglomerates resulting from the agglomeration.

3. Method according to claim 1 or 2,
**characterised in that** a sieve is used for the sizing of the resulting agglomerates.

4. Method according to claim 1,
**characterised in that** the particle size of the finely divided powdered medicament is smaller than 10µm and **in that** the size of the agglomerates after the agglomeration process is less than or equal to 2 mm.

5. Method according to claim 1,
**characterised in that** the finely divided powdered medicament is supplied to a screw feeder comprising twin concave screws having a pitch of the screws of about 2 - 20 mm, preferably 5 - 15 mm.

6. Method according to any of claims 1 to 5,
**characterised in that** it after the agglomeration in the screw feeder further comprises the step of sizing the agglomerated powder to give the agglomerates an uniform size.

7. Method according to claim 6,
**characterised in that** it after the spheronization comprises a further step of sizing and a further step of spheronizing.

8. Method according to claim 7,
**characterised in that** for the further sizing a further sieve is used and for the spheronizing a further tilted granulating container, preferably with one or more scrapers, is used.

9. Method according to any of the preceeding claims,
**characterised in that** the periphery speed of the granulating container preferably is 0.5 - 1.0 m/s.

10. Method according to any of the preceeding claims,
**characterised in that** the spheronization time preferebly is 2 - 20 min.

11. Apparatus for treatment of finely divided powdered medicament having a particle size smaller than 10 µm and poor flowing properties to cause the powdered medicament to, in a controlled manner, form small loose agglomerates or pellets which are free flowing and which are capable of breaking down to provide the finely divided medicament, wherein the apparatus comprises a screw feeder with at least two co-operating screws which are rotating, a spheronizing device for spheronizing the resulting agglomerates and a sizing device for sizing the agglomerates to obtain a uniform size of the final product.

12. Appartus according to claim 11,
**characterised in that** a tilted granulating container, preferably with one or more scrapers, is used to spheronize the agglomerates resulting from the agglomeration.

13. Appartus according to claim 11 or 12,
**characterised in that** a sieve is used for the sizing of the resulting agglomerates.

14. Apparatus according to claim 11,
**characterised in that** the screw feeder comprises twin-concave screws having a pitch of the screws of about 2 - 20 mm, preferably 5 - 15 mm.

15. Apparatus according to claim 11 or 12,
**characterised in that** it comprises a first further sieve, a second further sieve and a first further tilted granulating container, preferably with one or more scrapers.

16. Apparatus according to any of claims 11 to 15,
**characterised in that** the apertures of the sieves have a size between 0.2 - 2.0 mm, preferably between 0.3 -1.0 mm.

17. Use of an apparatus according to any of claims 11 to 16, to carry out a method according to any of claims 1 to 10.

18. Agglomerates manufactured according to the method as described in claims 1 to 10 using an appartus as described in claims 11 to 16 for use in a breath-actuated dry powder inhaler.

19. Agglomerates according to claim 18 wherein the inhaler is Turbuhaler® .

## Patentansprüche

1. Verfahren zur Behandlung eines feinteiligen pulverförmigen Arzneimittels mit einer Primärpartikelgröße unter 10 *µ*m und schlechten Fließeigenschaften zur kontrollierten Bildung von freifließenden Agglomeraten oder Pellets, die zerfallsfähig sind, so daß das feinteilige Arzneimittel bereitgestellt wird, wobei man bei dem Verfahren
a) das pulverförmige Medikament, bei dem die Substanz Partikelgrößen unter 10 *µ*m aufweist, dadurch agglomeriert, daß man das Material in einen Schneckenaufgeber einspeist und das Material durch den Schneckenaufgeber durchleitet, so daß man Agglomerate erhält,
b) die entstandenen Agglomerate sphäronisiert, so daß Agglomerate bereitgestellt werden, die sphärischer, dichter und kompakter als die bei der Agglomeration im Schneckenaufgeber erhaltenen Agglomerate sind, sowie
c) die Agglomerate klassiert, so daß man eine einheitliche Größe des Endprodukts erhält.

2. Verfahren nach Anspruch 1, **dadurch gekennzeichnet, daß** man zur Sphäronisierung der aus der Agglomeration stammenden Agglomerate einen geneigten Granulierbehälter, vorzugsweise mit einem oder mehreren Schabern, verwendet.

3. Verfahren nach Anspruch 1 oder 2, **dadurch gekennzeichnet, daß** man zur Klassierung der entstandenen Agglomerate ein Sieb verwendet.

4. Verfahren nach Anspruch 1, **dadurch gekennzeichnet, daß** die Partikelgröße des feinteiligen pulverförmigen Arzneimittels unter 10 *µ*m beträgt und daß die Größe der Agglomerate nach der Agglomeration 2 mm oder darunter beträgt.

5. Verfahren nach Anspruch 1, **dadurch gekennzeichnet, daß** mit dem feinteiligen pulverförmigen Arzneimittel ein Schneckenaufgeber beschickt wird, der konkave Doppelschnecken mit einer Ganghöhe von ungefähr 2 - 20 mm, vorzugsweise 5 - 15 mm, aufweist.

6. Verfahren nach einem der Ansprüche 1 bis 5,
**dadurch gekennzeichnet, daß** es nach der Agglomeration in dem Schneckenaufgeber weiterhin den Schritt der Klassierung des agglomerierten Pulvers umfaßt, so daß man die Agglomerate auf eine einheitliche Größe bringt.

7. Verfahren nach Anspruch 6, **dadurch gekennzeichnet, daß** es nach der Sphäronisierung einen weiteren Klassierungsschritt und einen weiteren Sphäronisierungsschritt umfaßt.

8. Verfahren nach Anspruch 7, **dadurch gekennzeichnet, daß** für die weitere Klassierung ein weiteres Sieb verwendet wird und daß für die Sphäronisierung ein weiterer geneigter Granulierbehälter, vorzugsweise mit einem oder mehreren Schabern, verwendet wird.

9. Verfahren nach einem der vorhergehenden Ansprüche, **dadurch gekennzeichnet, daß** die Umfangsgeschwindigkeit des Granulierbehälters vorzugsweise 0,5 - 1,0 m/s beträgt.

10. Verfahren nach einem der vorhergehenden Ansprüche, **dadurch gekennzeichnet, daß** die Sphäronisierungszeit vorzugsweise 2 - 20 Minuten beträgt.

11. Gerät zur Behandlung eines feinteiligen pulverförmigen Arzneimittels mit einer Partikelgröße unter 10 *µ*m und schlechten Fließeigenschaften für den Zweck, daß das pulverförmige Arzneimittel auf kontrollierte Weise zur Bildung von kleinen lockeren Agglomeraten oder Pellets gebracht wird, die freifließend und zerfallsfähig sind, so daß das feinteilige Arzneimittel bereitgestellt wird, wobei das Gerät einen Schneckenaufgeber mit mindestens zwei zusammenwirkenden rotierenden Schnecken, eine Sphäronisierungseinrichtung zur Sphäronisierung der entstandenen Agglomerate und eine Klassierungseinrichtung zur Klassierung der Agglomerate aufweist, so daß man zu einer einheitlichen Größe des Endprodukts gelangt.

12. Gerät nach Anspruch 11, **dadurch gekennzeichnet, daß** man zur Sphäronisierung der aus der Agglomeration stammenden Agglomerate einen geneigten Granulierbehälter, vorzugsweise mit einem oder mehreren Schabern, verwendet.

13. Gerät nach Anspruch 11 oder 12, **dadurch gekennzeichnet, daß** man zur Klassierung der entstandenen Agglomerate ein Sieb verwendet.

14. Gerät nach Anspruch 11, **dadurch gekennzeichnet, daß** der Schneckenaufgeber eine konkave Doppelschnecke mit einer Ganghöhe von ungefähr 2 - 20 mm, vorzugsweise 5 - 15 mm, umfaßt.

15. Gerät nach Anspruch 11 oder 12, **dadurch gekennzeichnet, daß** es ein erstes weiteres Sieb, ein zweites weiteres Sieb und einen ersten weiteren geneigten Granulierbehälter, vorzugsweise mit einem oder mehreren Schabern, umfaßt.

16. Gerät nach einem der Ansprüche 11 bis 15, **dadurch gekennzeichnet, daß** die Sieböffnungen eine Größe von 0,2 - 2,0 mm, vorzugsweise von 0,3 - 1,0 mm, aufweisen.

17. Verwendung eines Geräts nach einem der Ansprüche 11 bis 16 zur Durchführung eines Verfahrens nach einem der Ansprüche 1 bis 10.

18. Nach dem in Ansprüchen 1 bis 10 beschriebenen Verfahren mit einem wie in Ansprüchen 11 bis 16 beschriebenen Gerät hergestellte Agglomerate zur Verwendung in einem atembetätigten Trockenpulverinhalator.

19. Agglomerate nach Anspruch 18, wobei es sich bei dem Inhalator um den Turbuhaler® handelt.

## Revendications

1. Procédé de traitement d'un médicament en poudre finement divisé ayant une taille de particules primaires inférieure à 10 microns et des propriétés d'écoulement médiocres pour former, d'une manière contrôlée, des agglomérats ou des pellets qui sont à écoulement facile et qui sont capables de se désagréger pour fournir le médicament finement divisé, comprenant les étapes
a) d'agglomération du médicament en poudre ayant des tailles de particules inférieures à 10 microns de la substance en alimentant le matériau à un extracteur à vis, en laissant passer le matériau à travers l'extracteur à vis, en obtenant ainsi des agglomérats ;
b) de mise sous forme de sphères des agglomérats résultants de manière à fournir des agglomérats qui sont plus sphériques, plus denses et plus compacts que les agglomérats obtenus à partir du processus d'agglomération dans l'extracteur à vis,
c) de calibrage des agglomérats pour obtenir une taille uniforme du produit final.

2. Procédé selon la revendication 1, **caractérisé en ce que** l'on utilise un récipient de granulation incliné, de préférence avec un ou plusieurs racloirs, pour mettre sous forme de sphères les agglomérats résultant de l'agglomération.

3. Procédé selon la revendication 1 ou 2, **caractérisé en ce que** l'on utilise un tamis pour le calibrage des agglomérats résultants.

4. Procédé selon la revendication 1, **caractérisé en ce que** la taille des particules du médicament en poudre finement divisé est inférieure à 10 microns et **en ce que** la taille des agglomérats après le processus d'agglomération est inférieure ou égale à 2 mm.

5. Procédé selon la revendication 1, **caractérisé en ce que** le médicament en poudre finement divisé est fourni à un extracteur à vis comprenant des vis concaves jumelles ayant un pas de vis d'environ 2 - 20 mm, de préférence de 5 - 15 mm.

6. Procédé selon l'une quelconque des revendications 1 à 5, **caractérisé en ce qu'**il comprend, après l'agglomération dans l'extracteur à vis, l'étape de calibrage de la poudre agglomérée pour donner aux agglomérats une taille uniforme.

7. Procédé selon la revendication 6, **caractérisé en ce qu'**il comprend, après la mise sous forme de sphères, une autre étape de calibrage et une autre étape de mise sous forme de sphères.

8. Procédé selon la revendication 7, **caractérisé en ce que**, pour le calibrage supplémentaire, l'on utilise un tamis supplémentaire et **en ce que**, pour la mise sous forme de sphères, l'on utilise un récipient de granulation incliné supplémentaire, de préférence avec un ou plusieurs racloirs.

9. Procédé selon l'une quelconque des revendications précédentes, **caractérisé en ce que** la vitesse périphérique du récipient de granulation est de préférence de 0,5 - 1,0 m/s.

10. Procédé selon l'une quelconque des revendications précédentes, **caractérisé en ce que** la durée de mise sous forme de sphères est de préférence de 2 - 20 minutes.

11. Appareillage pour le traitement d'un médicament en poudre finement divisé, ayant une taille de particules inférieure à 10 microns et des propriétés d'écoulement médiocres pour former, d'une manière contrôlée, des agglomérats ou des pellets lâches, de petite taille, qui sont à écoulement facile et qui sont capables de se désagréger pour fournir le médicament finement divisé, **caractérisé en ce que** l'appareillage comprend un extracteur à vis ayant au moins deux vis rotatives, qui coopèrent, un dispositif de mise sous forme de sphères pour la mise sous forme de sphères des agglomérats résultants et un dispositif de calibrage pour le calibrage des agglomérats pour obtenir la taille uniforme du produit final.

12. Appareillage selon la revendication 11,
**caractérisé en ce que** l'on utilise un récipient de granulation incliné, de préférence avec un ou plusieurs racloirs, pour la mise sous forme de sphères des agglomérats résultants de l'agglomération.

13. Appareillage selon la revendication 11 ou 12,
**caractérisé en ce que** l'on utilise un tamis pour le calibrage des agglomérats résultants.

14. Appareillage selon la revendication 11,
**caractérisé en ce que** l'extracteur à vis comprend deux vis concaves jumelles ayant un pas de vis d'environ 2 - 20 mm, de préférence de 5 - 15 mm.

15. Appareillage selon la revendication 11 ou 12,
**caractérisé en ce qu'**il comprend, en outre, un premier tamis supplémentaire, un deuxième tamis supplémentaire et un premier récipient de granulation incliné supplémentaire, de préférence avec un ou plusieurs racloirs.

16. Appareillage selon l'une quelconque des revendications 11 à 15, **caractérisé en ce que** les ouvertures des tamis ont une taille comprise entre 0,2 et 2,0 mm, de préférence entre 0,3 et 1,0 mm.

17. Utilisation d'un appareillage selon l'une quelconque des revendications précédentes 11 à 16, pour exécuter un procédé conformément à l'une quelconque des revendications 1 à 10.

18. Agglomérats fabriqués conformément à la méthode telle qu'elle est décrite dans les revendications 1 à 10, en utilisant un appareillage tel qu'il est décrit dans les revendications 11 à 16, en vue de l'utilisation dans un inhalateur de poudre sèche à actionnement par la respiration.

19. Agglomérats selon la revendication 18, **caractérisé en ce que** l'inhalateur est l'unité Turbuhaler® .
